# EUROPEAN PATENT SPECIFICATION

(11) **EP 2 962 670 B1**
(45) Date of publication and mention of the grant of the patent: **21.02.2018**
(21) Application number: 15159472.8
(22) Date of filing: 17.03.2015
(51) Int. Cl.: A61F 5/48, A47C 31/10, A61G 7/02, A61G 9/00

(54) **BED PROTECTION DEVICE WITH ONE OR MORE RELEASABLE AND FASTENABLE WINGS**
BETTSCHUTZVORRICHTUNG MIT EINEM ODER MEHREREN LÖSBAREN UND FIXIERBAREN FLÜGELN
DISPOSITIF DE PROTECTION DE LIT AVEC UNE OU PLUSIEURS AILETTES POUVANT ÊTRE DÉTACHÉES ET FIXÉES

(30) Priority: 30.06.2014 EP 14002224
(43) Date of publication of application: 06.01.2016
(73) Proprietor: The Procter & Gamble Company, Cincinnati, OH 45202 (US)
(72) Inventor: Miralles, Jose, 08170 Montornès del Vallès (ES); Esquerra, Juan, 08170 Montornès del Vallès (ES)
(74) Representative: Chandrani, Vandita

(56) References cited:
- DE-A1-102006 028 834
- US-A- 5 221 273
- US-A1- 2006 264 863
- US-B1- 6 233 762

## Description

### FIELD OF THE INVENTION

The invention provides a bed protection device suitable for use over bedding beneath an incontinent person. The bed protection device comprises an absorbent pad and at least one wing. The one or more wings have one or more fasteners which are disposed at or adjacent to one or both of first and second lateral edges of the one or more wings. The one or more wings can be releasably engaged with the absorbent pad at or adjacent to a first and/or second lateral edge of the absorbent pad. Moreover, the invention provides a package comprising one or more absorbent pads and one or more wings able to releasably engage with each absorbent pad of the package.

### BACKGROUND OF THE INVENTION

Bed protection devices are used in hospitals, long term care facilities, and private home to protect bedding, mattresses against any bodily exudates that might deposit on the bed, penetrate and damage the mattress. Bed protection devices have been developed and placed on top of the bed directly beneath the patient disposed on the bed. The bed protection device can consist of an absorbent pad comprising a liquid permeable topsheet, a liquid impermeable backsheet and an absorbent layer disposed between the topsheet and the backsheet, see for example U.S. Patent application 4,097,943.

A bed protection device may comprise in addition to a center pad comprising an absorbent layer, an additional layer of material which is twice as long as the center pad. The center pad is disposed at the center of the additional layer such that the bed protection device comprises a first and second wing. Alternatively, the first and second wing may be made of two separate layers and may be directly attached to the center pad. The bed protection device can be secured to the bed or mattress via the first and second wing, see for example U.K. Patent application GB 2501561.

In both bed protection device embodiments, when the bed protection device has been soiled, the bed protection device can be replaced and disposed of when required without the necessity of changing and laundering textile sheets of the bed. However, when the bed protection device comprises one or more wings, the bed protection device is discarded with the one or more wings.

US6233762 relates to a device and a method to prevent bed sheet, mattress, or bed soiling by body fluid. The device contains a waterproof panel removably attached to a panel anchor. Two tuck panels, one attached to either side of the panel anchor, are used to stabilize the stain prevention device, preventing it from being moved around on the bed. The tuck panels are tucked underneath the mattress to stabilize the stain prevention device.

US2006264863 is considered to represent the closest prior art and relates to a one piece disposable, biodegradable, form fitting and absorbent protective article fabricated with a biodegradable non-woven hydrophilic top sheet, a fluid acquisition and distribution layer comprised of SAP, SAP/thermoplastic fiber blends or SAP/cellulosic fiber blends, a back sheet of a biodegradable fluid impervious thermoplastic film, and a contour conforming side panel capable of stretching both transversely and longitudinally and is secured with elastic banding or draw string. The entire article is fabricated by means of heat sealed contact points and hot melt adhesives.

It will be beneficial to provide a more sustainable bed protection device.

It will be also advantageous to provide a bed protection device wherein one can place the adequate absorbent pad according to the usage conditions of the bed protection device. There is thus further a need to provide a more adaptable bed protection device providing different types of absorbent layers, or different types of wings in order to accommodate to more diverse usage conditions or to fit different sizes of beds or other surfaces to be protected from any liquid bodily exudate contaminations.

### SUMMARY OF THE INVENTION

A bed protection device is provided and comprises an absorbent pad having first and second lateral edges. The absorbent pad comprises a liquid permeable topsheet, a liquid impermeable backsheet and an absorbent layer located between the topsheet and the backsheet. The bed protection device further comprises at least one wing. The one or more wings is made of a material selected from the group consisting of a woven fabric, a nonwoven web, a film-nonwoven fabric laminate, a tissue and combinations thereof. The one or more wings have first and second lateral edges. One or more fasteners are disposed at or adjacent to each of the first and second lateral edges of the absorbent pad on the topsheet or the backsheet and/or one or more fasteners are disposed at or adjacent to one or both of the first and second lateral edges of the one or more wings, such that the one or more wings are able to be releasably engaged with the absorbent pad at or adjacent to the first and/or second lateral edge of the absorbent pad on the topsheet or the backsheet by the one or more fasteners.

A package is provided and comprises:
- One or more absorbent pads, each having a first and second lateral edges and comprising a liquid permeable topsheet, a liquid impermeable backsheet and an absorbent layer located between the topsheet and the backsheet;
- One or more wings, each having a first and a second lateral edges, the one or more wings being material selected from the group consisting of a woven fabric, a nonwoven web, a film-nonwoven fabric laminate, a tissue and combinations thereof;
- One or more fasteners disposed at or adjacent to each of the first and second lateral edges of the absorbent pad on the topsheet or the backsheet and/or one or more fasteners disposed at or adjacent to one or both of the first and second lateral edges of the one or more wings.

The one or more wings are able to be releasably engaged with the absorbent pad at or adjacent to the first and/or second lateral edge of the absorbent pad on the topsheet or the backsheet by the one or more fasteners.

### BRIEF DESCRIPTION OF THE DRAWINGS

While the specification concludes with claims particularly pointing out and distinctly claiming the present invention, it is believed that the same will be better understood from the following description read in conjunction with the accompanying drawings in which:
Fig. 1 is a plan view of a bed protection device with a portion of a topsheet and a wing cut away to show an absorbent layer and a backsheet, both of which underlie the topsheet cut-away;
Fig. 2 is a schematic sectional view taken along the line 2-2 of Fig. 1;
Fig. 3 is a perspective view of the bed protection device of Fig. 1 placed on the bed and secured to the mattress;
Fig. 4 is a schematic sectional view of Fig. 3 taken along the line 4-4 of Fig. 3;
Fig. 5 is a schematic sectional view according to one non-limiting embodiment of the present invention;
Fig. 6 is a perspective view of a bed protection device with only one wing according to one non-limiting embodiment of the present invention;
Fig. 7 is a perspective view of the bed protection device of Fig. 6 placed on the bed and secured to the mattress;
Fig. 8 is a schematic sectional view of Fig. 7 taken along the line 8-8 of Fig. 7;
Fig. 9 is a schematic sectional view according to one non-limiting embodiment of the present invention;
Fig. 10 is a schematic sectional view according to one non-limiting embodiment of the present invention;
Fig. 11 is a plan view of a wing having on the first lateral edge a stripe of hook fasteners;
Fig. 12 is a zoomed view of a portion of the stripe of hook fasteners of Fig. 11;
Fig. 13 is fragmentary perspective view, partially broken away, of a first lateral edge of a wing to which a tape tab has been applied;
Fig. 14A-14D are plan views of a wing illustrating suitable fasteners configurations.

### DETAILED DESCRIPTION OF THE INVENTION

### Definition of terms

The term "bed protection device" as used herein refers to a disposable product for an adult incontinent person but also for a baby and which is placed beneath the body of the wearer to absorb and contain the various liquid bodily exudates discharged from the body. A bed protection device comprises a disposable absorbent pad which has a topsheet, a backsheet, an absorbent layer and optionally one or more wings, with the absorbent layer normally placed between the topsheet and the backsheet. The one or more wings may be washable or may be disposable. The bed protection device can be put over a mattress but also over any types of surface to be protected against any liquid bodily exudates.

The term "wing" as used herein is a panel made of a material selected from the group consisting of a woven fabric, a nonwoven web, a film-nonwoven fabric laminate, a tissue and combinations thereof. A nonwoven web or tissue can show a good compatibility with the skin. The wing may have a width from 100 mm to 2000 mm along the longitudinal central axis of the bed protection device and a length from 100 mm to 1200 mm along the transverse central axis of the bed protection device. Each wing 40 may have a width not lower than 50% of the width of the absorbent pad 11. The wing may be made of a non-elastic material.

The term "non-extensible wing" as used herein refers to a wing which, upon application of a force, elongates beyond its original length by less than 20% if subjected to the following test. The length of the wing is measured in a direction substantially parallel to the transverse axis of the bed protecting device:
A rectangular piece of the wing having a width of 2.54 cm and a length of 25.4 cm is maintained in a vertical position by holding the piece along its upper 2.54 cm wide edge along its complete width. A force of 10 N is applied onto the opposite lower edge along the complete width of the material for 1 minute (at 25°C and 50% relative humidity; samples should be preconditioned at these temperature and humidity conditions for 2 hours prior to testing). Immediately after one minute, the length of the piece is measured while the force is still applied and the degree of elongation is calculated by subtracting the initial length (25.4 cm) from the length measured after one minute.

The term "non-elastic wing" as used herein refers to a wing which does not recover by more than 20% if subjected to the following test, which is to be carried out immediately subsequent to the test on "non-extensibility" set out above.

Immediately after the length of the rectangular piece of wing has been measured while the 10N force is still applied, the force is removed and the piece is laid down flat on a table for 5 minutes (at 25°C and 50% relative humidity) to be able to recover. Immediately after 5 minutes, the length of the piece is measured again and the degree of elongation is calculated by subtracting the initial length (25.4 cm) from the length after 5 minutes.

The elongation after one minute while the force has been applied (as measured with respect to "non-extensibility") is compared to the elongation after the piece has been laid down flat on a table for 5 minutes: If the elongation does not recover by more than 20%, the material is considered to be "non-elastic".

The term "disposable" as used herein means when referring to an absorbent pad that the absorbent pad is intended to be discarded after a single use. When referring to a wing, the term "disposable" means that the wing can be used once or several times (such as up to 10 times) before being discarded.

The term "absorbent layer" as used herein refers to a component, which is placed or is intended to be placed within a bed protection device and which comprises an absorbent material which may be enclosed in a core wrap. The absorbent layer is typically the component of an absorbent pad of the bed protection device which comprises all, or at least the majority of an absorbent material, preferably fibrous in nature and/or with a superabsorbent material and has the highest absorbent capacity of all the components of the bed protection device.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essential of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "advantageously" and the likes also qualify features which are not intended to limit the scope of the claims unless specifically indicated to do so.

The term "cellulosic fiber" as used herein refers to natural fibers which typically are wood pulp fibers. Applicable wood pulps include chemical pulps, such as Kraft, sulfite, and sulfate pulps, as well as mechanical pulps including, for example, groundwood, thermomechanical pulp and chemically modified thermomechanical pulp. Pulps derived from both deciduous trees (hereinafter, also referred to as "hardwood") and coniferous trees (hereinafter, also referred to as "softwood") may be utilized. The hardwood and softwood fibers can be blended, or alternatively, can be deposited in layers to provide a stratified web.

The term "fastener" refers to a device permanently attached at a first layer which is able to releasably engage a second layer either in a mechanical or chemical way.

The term "adjacent to a first and/or second lateral edge of a wing" as used herein means that one or more fasteners are disposed within an area spaced inboard from a first and/or second lateral edge of the wing. The area has a width which is from 1 to 30% of a width of the wing. If the one or more fasteners are provided on a tape tab, the one or more fasteners are disposed laterally outboard beyond the first and/or second lateral edge of the wing.

The term "adjacent to a first and/or second lateral edge of an absorbent pad" as used herein means that one or more fasteners are disposed within an area spaced inboard from a first and/or second lateral edge of the absorbent pad. The area has a width which is from 1 to 30% of a width of the absorbent pad. If the one or more fasteners are provided on a tape tab, the one or more fasteners are disposed laterally outboard beyond the first and/or second lateral edge of the absorbent pad.

The term "nonwoven web" as used herein refers to a manufactured material, web, sheet or batt of directionally or randomly oriented fibers, bonded by friction, and/or cohesion and/or adhesion, excluding paper and products which are woven, knitted, tufted, stitch-bonded, incorporating binding yarns or filaments, or felted by wet milling, whether or not additionally needled. The fibers may be of natural or man-made origin. The fibers may be staple or continuous filaments or be formed in situ. The porous, fibrous structure of a nonwoven may be configured to be liquid permeable or impermeable, as desired.

The term "tissue" as used herein refers to sheets of paper made by a process which may comprise the steps of forming an aqueous papermaking furnish, depositing this furnish on a foraminous surface, such as a Fourdrinier wire, and removing the water from the furnish (e.g. by gravity or vacuum-assisted drainage), forming an embryonic web, transferring the embryonic web from the forming surface to a transfer surface. The web is then transferred to a fabric upon which it is through air dried to a final dryness after which it is wound upon a reel.

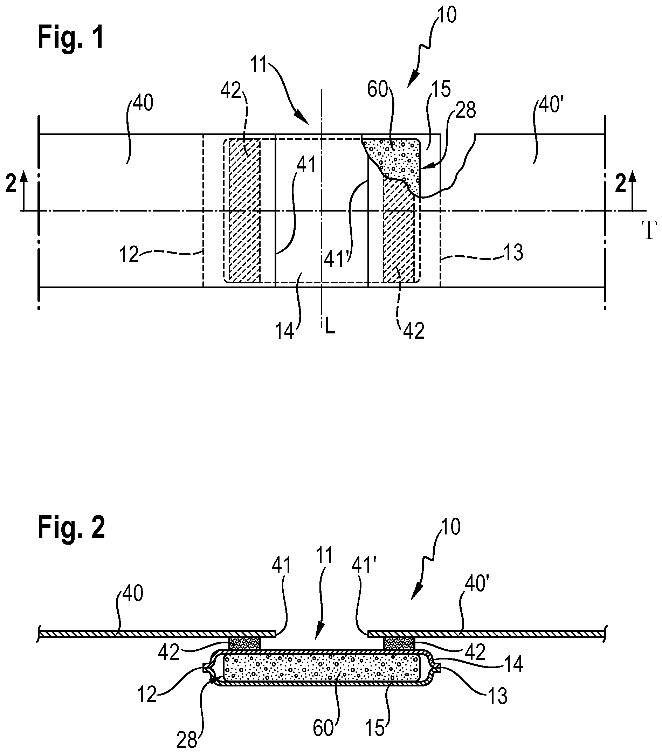

Fig. 1 and 2 show a bed protection device 10 which comprises an absorbent pad 11. The absorbent pad 11 has a longitudinal central axis L and a transverse central axis T. The longitudinal axis L of the absorbent pad 11 is the imaginary line separating the absorbent pad 11 along its length in two halves. The transverse central axis T of the absorbent pad 11 is the imagery line perpendicular to the longitudinal axis L in the plane of the absorbent pad 11 and going through the middle of the length of the absorbent pad 11. The absorbent pad 11 has first and second lateral edges (12, 13). The first and second lateral edges (12, 13) of the absorbent pad 11 are the lateral edges of an absorbent pad 11 running generally parallel to the longitudinal axis L of the absorbent pad 11. The absorbent pad 11 comprises a liquid permeable topsheet 14, a liquid impermeable backsheet 15 and an absorbent layer 28. The absorbent layer 28 is located between the topsheet 14 and the backsheet 15. The absorbent pad 11 may be disposable.

The absorbent pad 11 may have a width from 100 mm to 1200 mm or from 300 mm to 1200 mm along the longitudinal central axis L of the bed protection device 10 and a length from 100 mm to 1000 mm or from 300 mm to 1000 mm along the transverse axis T of the bed protection device 10.

The bed protection device 10 may have any suitable length and width required for an adult incontinent person or for a baby. Hence, generally the bed protection device 10 to be disposed beneath an adult incontinent person or a baby will have a total width from 100 mm to 2000 mm or from 300 mm to 1500 mm or from 500 mm to 1000 mm along the longitudinal central axis L of the bed protection device 10 and a total length from 200 mm to 2200 mm or from 500 mm to 1500 mm along the transverse central axis T of the bed protection device 10.

When a bed protection device 10 is placed beneath an adult incontinent person in hospitals, the mattress of the bed has to be sufficiently protected from any liquid bodily exudate contaminations.

The bed protection device 10 is also useful to assist the caregiver for toilet-training his child when it is desired to not use diapers any more. The bed protection device 10 may thus be used in that case to cover the mattress for temporary protection. Alternatively, one may consider using the bed protection device 10 as a device to isolate any suitable surface, such as a table on which the baby is changed by the caregivers. The suitable surface may also include, but not limited to: bed sheets, mattresses, bet mats, diaper changing tables, sofas, chairs, seats and floors.

The liquid permeable topsheet 14 may include a woven fabric, a nonwoven web, a polymer film, a film-nonwoven fabric laminate or the like, as well as combinations thereof. A nonwoven web useable as topsheet 14 or comprised by a topsheet 14 may include, for example, an airlaid nonwoven web, a spunbond nonwoven web, a meltblown nonwoven web, a bonded- carded web, a hydroentangled nonwoven web, a spunlace web or the like, as well as combinations thereof. The topsheet 14 may be made of a nonwoven web having a basis weight from 5 to 50 gsm or from 10 to 20 gsm.

Other conventional examples of suitable materials for constructing the topsheet 14 may include rayon, bonded-carded webs of polyester, polypropylene, polyethylene, nylon, or other heat-bondable fibers, finely perforated film webs, net-like materials, and the like, as well as combinations thereof. When the topsheet 14 is a film or a film laminate, the film should be fluid permeable. The topsheet 14 may e.g. be rendered fluid permeable by aperturing. Alternatively, the topsheet 14 may be made of a composite material comprising a polymer and a nonwoven fabric material.

As shown in Fig. 1, the topsheet 14 extends beyond the absorbent layer 28. The topsheet 14 may be bonded to the backsheet 15, by any conventional means such as adhesives, heat/pressure sealing, ultrasonic bonding, or any other means or methods as are known in the art. It is contemplated, however, that the topsheet 14 can have the same extent as the absorbent layer 28 and/or backsheet 15 or can have an extent less that the absorbent layer 28 and/or backsheet 15.

The liquid impermeable backsheet 15 may be a polymeric film, a woven fabric, a nonwoven web or the like, as well as combinations or composites thereof. For example, the liquid impermeable backsheet 15 may include a polymer film laminated to a woven or nonwoven web. The polymer film can be composed of polyethylene, polypropylene, polyester, silicone or the like, as well as combinations thereof. For example, the backsheet 15 may be spunbond/meltblown/spunbond nonwoven treated with a polyethylene coating. Additionally, the backsheet 15 may be embossed, have a printed design, have a printed message to the consumer, and/or may be at least partially colored. In case that the backsheet 15 is made of a permeable material such as a nonwoven web, the absorbent pad 11 may include a barrier layer disposed between the absorbent layer 28 and the nonwoven backsheet.

The backsheet 15 and/or the topsheet 14 may be attached to the absorbent layer 28 in order to keep the absorbent layer 28 in place between the topsheet 14 and backsheet 15 when the incontinent person is moving on the bed protection device 10.

The absorbent layer 28 is configured to absorb the liquid bodily exudates including, but not limited to urine that passes through the topsheet 14. Suitable absorbent materials 60 which can be used to form the absorbent layer 28 include those absorbent materials 60 conventionally used in an absorbent pad and include absorbent materials, such as, for example, superabsorbent material, cellulose fibers and/or synthetic fibers. Synthetic fibers may be meltblown polymers such as polyester or polypropylene. Another suitable absorbent material is coform which is a meltblown air-formed combination of meltblown polymers, such as polypropylene, and cellulosic fibers.

The absorbent layer 28 may include a superabsorbent material 60, in addition to or in place of the cellulosic fibers. The superabsorbent material 60 may increase the ability of the absorbent layer 28 to absorb a large amount of fluid in relation to its own weight. Generally stated, the superabsorbent material 60 may be a water-swellable, generally water-insoluble, hydrogel-forming polymeric absorbent material, which is capable of absorbing at least about 15, suitably about 30, and possibly about 60 times or more its weight in physiological saline (e.g., saline with 0.9 wt% NaCl).

The superabsorbent material 60 may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like.

Other suitable superabsorbent material 60 may include hydrolyzed acrylonitrile grafted starch, acrylic acid grafted starch, and isobutylene maleic anhydride copolymers and mixtures thereof.

The absorbent layer 28 may comprise from 10 to 200 gsm or from 20 to 100 gsm of absorbent material 60.

The bed protection device 10 comprises at least one wing 40.

The one or more wings (40, 40') have first and second lateral edges (41, 43). The one or more wings (40, 40') may be disposable and/or laundered. The one or more wings (40, 40') have a longitudinal axis Lw and a transverse axis Tw. The longitudinal axis Lw of the one or more wings (40, 40') is the imaginary line separating the one or more wings (40, 40') along their length in two halves. The transverse central axis Tw of the one or more wings (40, 40') is the imagery line perpendicular to the longitudinal axis Lw in the plane of the one or more wings (40, 40') and going through the middle of the length of the one or more wings (40, 40'). The first and second lateral edges (41, 43) of the one or more wings (40, 40') are the edges running generally parallel to the longitudinal axis Lw. In the bed protection device 10, the longitudinal axis L of the absorbent pad 11 is substantially parallel to the longitudinal axis Lw of the one or more wings (40, 40').

When the bed protection device 10 comprises two separate wings (40, 40'), each wing (40, 40') may have a width from 100 mm to 2000 mm or 300 mm to 1200 mm along the longitudinal central axis L of the bed protection device 10 and a length from 100 mm to 1200 mm or 300 mm to 1000 mm along the transverse central axis T of the bed protection device 10.

Each wing (40, 40') may have dimensions in length and width that differ from the ones of the absorbent pad 11. This can provide different sizes of wings (40, 40') for different sizes of absorbent pads 11. Depending on the size of the mattress, it may be useful to provide different sizes of wings (40, 40').

Each wing (40, 40') may have a width not lower than 50% of the width of the absorbent pad 11.

Alternatively, each wing (40, 40') may have the same dimensions in length and width than the absorbent pad 11.

The absorbent pad 11 of the bed protection device 10 may comprise a central area which is an area around the intersection of the longitudinal central axis L and the transverse central axis T of the bed protection device 10.

When the width of each wing (40, 40') is similar to the width of the absorbent pad 11 of the bed protection device 10, even if the central area of the absorbent pad 11 is not secured to the mattress, the absorbent pad 11 does not become wrinkled. Indeed, when the wings (40, 40') are tucked under the mattress, the wings (40, 40') can provide enough tension to the absorbent pad 11 to prevent the formation of wrinkles on the absorbent pad 11. Avoiding or reducing wrinkles in the absorbent pad 11 can help promoting comfort and also preventing the formation of bed sores.

Alternatively, the bed protecting device 10 may comprise two pairs of two wings wherein the first wings of the respective pair of wings may be releasably engaged at or adjacent to the first lateral edge 12 of the absorbent pad 11 on the topsheet 14 or the backsheet 15; and wherein the second wings of the respective pair of wings may be releasably engaged at or adjacent to the second lateral edge 13 of the absorbent pad 11 on the topsheet 14 or the backsheet 15. Two pairs of wings can facilitate the placement of the absorbent pad 11, e.g. if a bed 30 is relatively large. Indeed each pair of wings can be placed at each extremity of the bed 30 when the bed protecting device 10 is placed on top of the bed 30. The placement of each pair of wings at each extremity of the bed can be more convenient than at a center of the bed 30 where this is more difficult to lift the mattress for tucking each wing under the mattress 31.

The one or more wings (40, 40') is made of a material selected from the group consisting of a woven fabric, a nonwoven web, a film-nonwoven fabric laminate, a tissue and combinations thereof With smaller absorbent pads 11, the skin of the consumer may be in contact with the one or more wings (40, 40'). Hence, the one or more wings (40, 40') made of a nonwoven web or tissue may be preferred for better compatibility with the skin.

The material of the wings may have hydrophobic properties in order to avoid the mattress getting wet.

Alternatively, the one or more wings 40 may be formed from a textile material having water penetration resistance, but still providing good heat transfer characteristics and launderability. Suitable textile materials may be woven fabrics formed of cotton and/or synthetic fibers. Hence, the one or more wings 40 may be made of a breathable material in order to prevent the person laid down on the bed protection device sweating. The one or more wings 40 may have a basis weight from 2 to 70 gsm or from 10 to 30 gsm.

One or more fasteners (42, 44, 44', 45) are disposed at or adjacent to each of the first and second lateral edges (12, 13) of the absorbent pad 11 and/or at or adjacent to one or both of the first and second lateral edges (41, 43) of the one or more wings (40, 40').

Hence, the one or more wings (40, 40') are able to be releasably engaged with the absorbent pad 11 at or adjacent to the first and/or second lateral edges (12, 13) of the absorbent pad 11.

The bed protection device 10 may comprise a first and a second wings (40, 40'), as shown in Fig. 1 and Fig. 2. One or more fasteners 42 may be disposed at or adjacent to the first lateral edge 41 of the first wing 40. The first wing 40 may be thus able to be releasably engaged with the absorbent pad 11 at or adjacent to the first lateral edge 12 of the absorbent pad 11. One or more fasteners 42 may be disposed at or adjacent to the first lateral edge 41' of the second wing 40'. The second wing 40' may thus be able to be releasably engaged with the absorbent pad 11 at or adjacent to the second lateral edge 13 of the absorbent pad 11.

The first wing 40 may be able to be releasably engaged to the topsheet 14 of the absorbent pad 11 at or adjacent to the first lateral edge 12 of the absorbent pad 11. The second wing 40' may be able to be releasably engaged to the topsheet 14 of the absorbent pad 11 at or adjacent to the second lateral edge 13 of the absorbent pad 11, as shown in Fig. 1 and 2.

Alternatively, the first wing 40 may be able to be releasably engaged to the backsheet 15 of the absorbent pad 11 at or adjacent to the first lateral edge 12 of the absorbent pad 11. The second wing 40' may be able to be releasably engaged to the backsheet 15 of the absorbent pad 11 at or adjacent to the second lateral edge 13 of the absorbent pad 11.

As set out above, the backsheet material 15 may be a polyethylene film, which may be covered by a nonwoven material. Hence, the first and second wing (40, 40') may be able to be releasably engaged on the nonwoven material of the backsheet 15 of the absorbent pad 11.

The first and second wings (40, 40') may be thus releasably engaged to the topsheet 14 or to the backsheet 15 of the absorbent pad 11. Hence, the consumer can choose which option fits best to his needs.

When first and second wings (40, 40') are attached to the backsheet 15 of the absorbent pad 11, the likelihood of contamination of the re-usable wings by any fluid from the absorbent pad 11 can be reduced. Also, this can provide a comfortable bed protection device 10, since the one or more fasteners 42 of first and second wings (40, 40') are now positioned under the absorbent pad 11.

The one or more fasteners 42 when releasably engaged with the backsheet 15 of the absorbent pad 11 can be less exposed to fluid which can help increasing their life-time for re-use of the releasable wings (40, 40'). Especially in the case when the one or more fasteners 42 are made of a pressure-sensitive adhesive and when the one or more fasteners 42 are less in contact with the fluid from the absorbent pad 11, it is unlikely that the pressure-sensitive adhesive may tend to flow on the linen of the mattress.

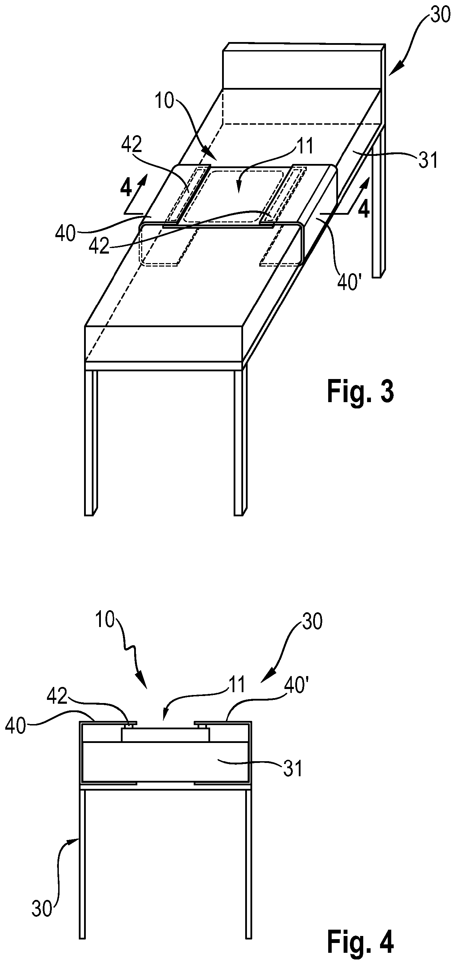

As illustrated in Fig. 3 and Fig. 4, a bed 30 comprises a mattress 31 and the bed protection device 10. The first and second wings (40, 40') of the bed protection device 10 which are releasably engaged with the absorbent pad 11 at or adjacent to the respective first and second lateral edges (12, 13) of the absorbent pad 11 have been tucked under the mattress 31. The first and second wings (40, 40') of the bed protection device 10 allow securing the bed protection device 10 to the mattress. Additionally, as the first and second wings (40, 40') of the bed protection device 10 can be detached from the absorbent pad 11, the soiled absorbent pad 11 can be readily disposed. The first and second wings (40, 40') can then be reused several times without a need to be laundered in-between. The first and second wings (40, 40') can remain tucked under the mattress 31 when changing the soiled absorbent pad 11, which provides a more flexible bed protection device 10. The first and second wings (40, 40') may be laundered when the first and second wings 40 are made of an appropriate material as set out above.

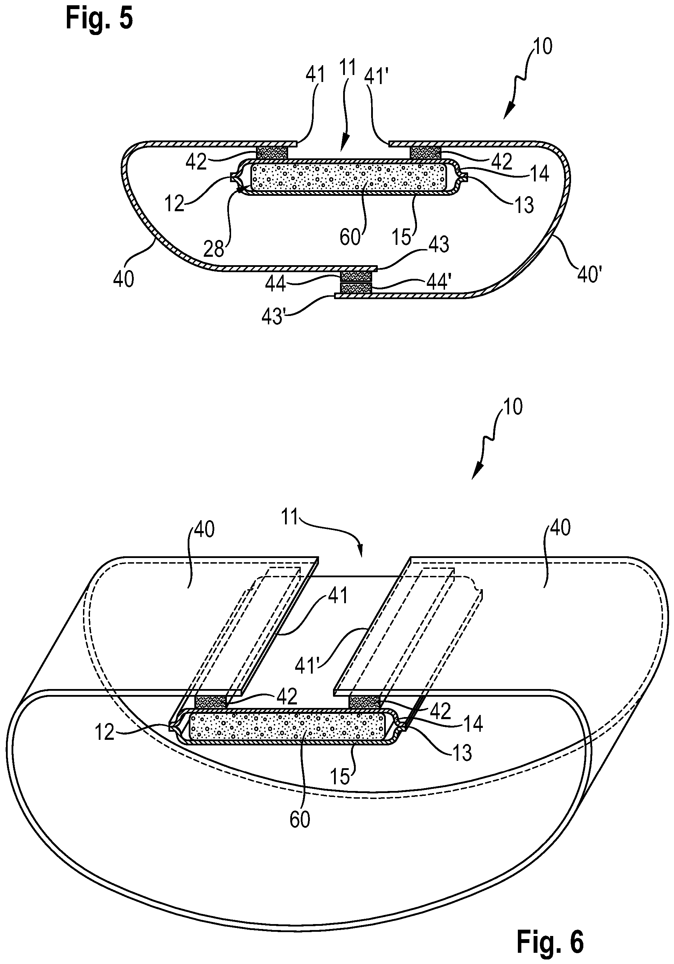

One or more fasteners 44 which are disposed at or adjacent to the second lateral edge 43 of the first wing 40 may be able to be releasably engaged with one or more fasteners 44' disposed at or adjacent to the second lateral edge 43' of the second wing 40', as shown in Fig. 5. Prior to or after positioning the first and second wing (40, 40') under the mattress 31, the first and second wings (40, 40') can be releasably attached to each other at their respective second lateral edges (43, 43'). The first lateral edges (41, 41') of each of the first and second wings (40, 40') are then engaged with the respective first and second lateral edges (12, 13) of the absorbent pad 11. Once in place, the bed protection device 10 may be relatively tightly secured around the mattress 31 of the bed 30. This optional feature may be of interest in case when the person laid down on the bed may move and displace the bed protection device 10 with the first and second wings (40, 40').

Alternatively, the bed protection device 10 may comprise only one wing 40, as shown in Fig. 6. One or more fasteners 42 disposed at or adjacent to the first lateral edge 41 of the only one wing may 40 be able to be releasably engaged with the absorbent pad 11 at or adjacent to the first lateral edge 12 of the absorbent pad 11. One or more fasteners 42 disposed at or adjacent to the second lateral edge 41' of the only one wing 40 may be able to be releasably engaged with the absorbent pad 11 at or adjacent to the second lateral edge 13 of the absorbent pad 11.

When the bed protection device 10 comprises only one wing 40, the only one wing 40 may have a width from 100 mm to 2000 mm or from 600 mm to 2000 mm along the longitudinal central axis L of the bed protection device 10 and a length from 200 mm to 3500 mm or from 600 mm to 2200 mm along the transverse central axis T of the bed protection device 10.

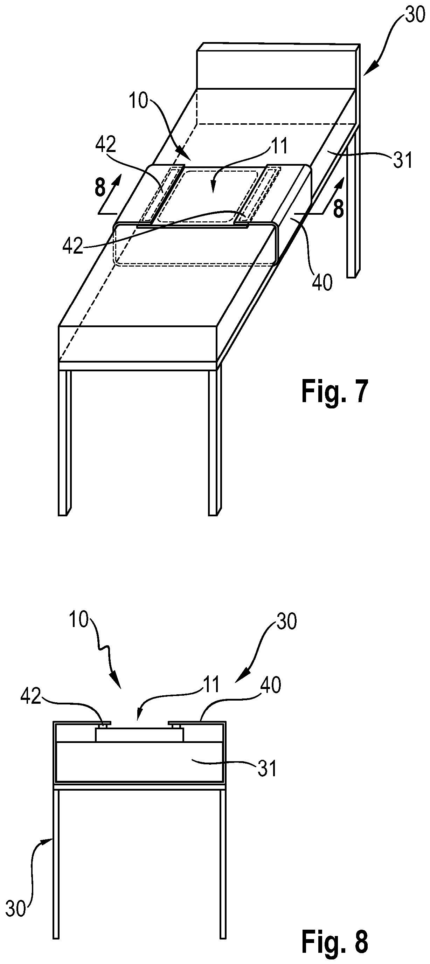

As illustrated in Fig. 7 and Fig. 8, the bed 30 comprises the bed protection device 10 having only one wing 40. The only one wing 40 of the bed protection device 10 which is releasably engaged with the absorbent pad 11 at or adjacent to the respective first and second lateral edges (12, 13) of the absorbent pad 11 is supplemented below the mattress 31. The bed protection device 10 surrounds the mattress 31. Hence, the only one wing 10 of the bed protection device 10 increases the stability of the bed protection device 10 to the mattress 31 in case the incontinent person is moving on the mattress 31 overnight. The absorbent pad 11 can also be changed without removing the only one wing 40 from the bed 30. Such bed protection device 10 may be also suitable to secure any types of surface like a diaper changing table or wheelchair.

Additionally, the absorbent pad 11 may comprise one or more fasteners 45 which are disposed at or adjacent to each of the first and second lateral edges (12, 13) of the absorbent pad 11. The one or more fasteners 45 at each of the first and second lateral edges (12, 13) of the absorbent pad 11 may be provided either on the topsheet 14 or the backsheet 15 or between the topsheet 14 and backsheet 15.

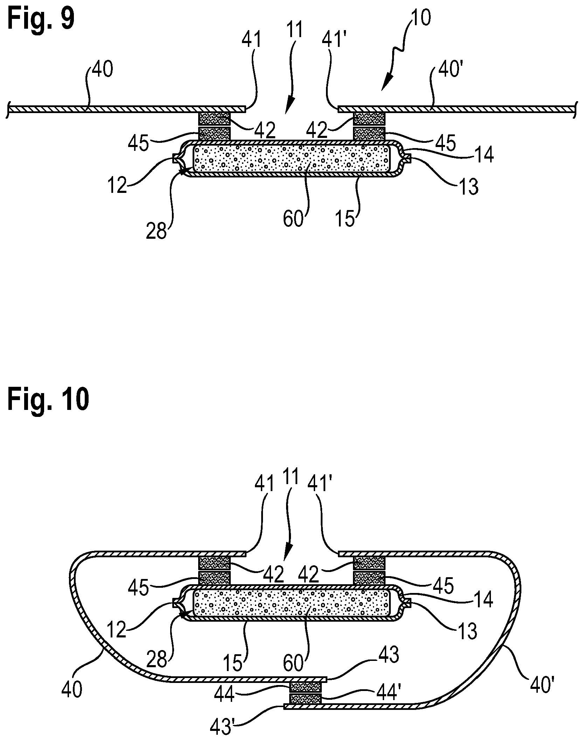

When the bed protection device 10 comprises first and second wings (40, 40'), the first wing 40 may be able to be releasably engaged with the absorbent pad 11 at or adjacent to the first lateral edge 12 of the absorbent pad 11. The second wing 40 may be able to be releasably engaged with the absorbent pad 11 at or adjacent to the second lateral edge 13 of the absorbent pad 11, as shown in Fig. 9 or another example in Fig. 10.

The only one wing 40 may be also able to be releasably engaged with the absorbent pad 11 at or adjacent the first and second lateral edges (12, 13) of the absorbent pad 11.

When the absorbent pad 11 further comprises one or more fasteners 45 which are disposed at or adjacent to each of the first and second lateral edges (12, 13) of the absorbent pad 11, the one or more fasteners 45 on the absorbent pad 11 provides a complementary fastener to the one or more fasteners 42 of the first and second wings (40, 40'), or the only one wing 40 (such as a hook fastener and a complimentary loop fastener). Hence, the reliable fastening of the absorbent pad 11 with the first and second wings (40, 40') or the only one wing 40 is optimized. The bed protection device 10 may be firmly secured to the mattress 31.

One or more fasteners 45 of any of the first and second lateral edges (12, 13) of the absorbent pad 11 may be complementary to one or more fasteners 42 which are disposed at or adjacent to the first or second lateral edges (41, 43) of the one or more wings (40, 40'). When the one or more fasteners 45 on the absorbent pad 11 are complementary to the one or more fasteners 42 of the one or more wings (40, 40'), the strength of the releasable attachment is enhanced. It is unlikely that the one or more wings (40, 40') are released without the intervention of a caregiver.

One or more fasteners (42, 45) may be mechanical or chemical fasteners. The mechanical fastener may be a hook fastener or an interlocking fastener (such as a button which can engage with a complimentary button hole). A hook fastener may be releasably engageable with e.g. a nonwoven web (comprising a plurality of fibers forming fibrous loops) or with a complimentary loop fastener (such as a knitted or nonwoven web).

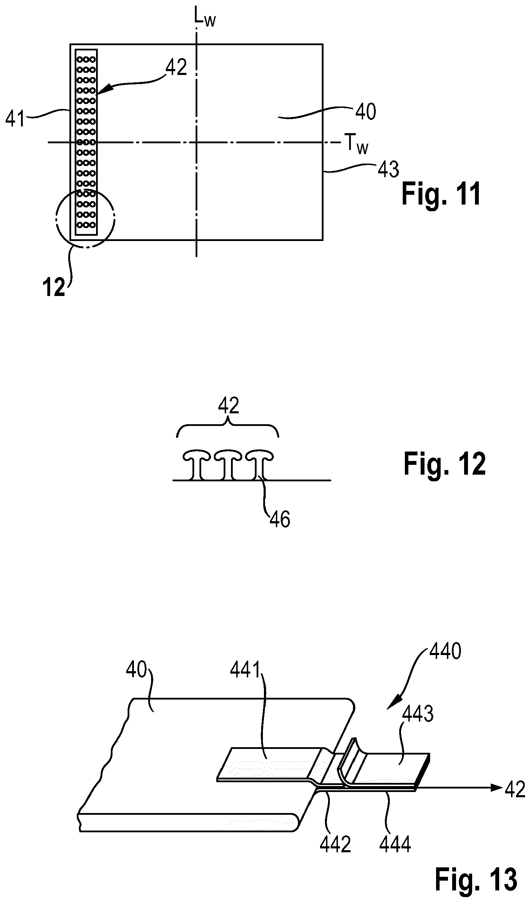

One or more fasteners (42, 45) may be a hook or a loop fastener. As depicted in Figs. 11 and 12, a hook fastener 42 may comprise a plurality of resiliently flexible spaced hook members 46.

One or more loop fasteners 45 may be disposed at or adjacent to each of the first and second lateral edges (12, 13) of the absorbent pad 11. When one or more hook fasteners 42 are disposed at or adjacent to the first lateral edge (41, 41') of the first and second wings (40, 40'), the first and second wings (40, 40') may be releasably engaged with the absorbent pad 11. Indeed, the hook fasteners 42 of the first and second wings (40, 40') may engage with the loop fasteners 45 of the absorbent pad 11.

The loop fastener may be the fibrous loops of a nonwoven web constituting the topsheet 14 of the absorbent pad 11 or the fibrous loops of a nonwoven web constituting the backsheet 15 of the absorbent pad 11. Alternatively, the loop fastener may be provided by a knitted fabric.

The mechanical fasteners may be interlocking fasteners. An interlocking fastener may comprise a tap and a slot member. For instance, a tap member may be disposed at or adjacent the first lateral edge (41, 41') of one or more wings (40, 40'). The slot member may be disposed at the first and second lateral edges (12, 13) of the absorbent pad 11. The slot member is a portion of the interlocking fastener through which the tab member of the one or more wings (40, 40') is passed in order to engage of fasten the one or more wings (40, 40') to the absorbent pad 11.

A chemical fastener may be pressure sensitive adhesive. Pressure-sensitive adhesives are typically formulated to adhere to a surface at ambient temperature by briefly applying light pressure alone. Pressure-sensitive adhesives may be emulsion, solvent, and hot melt pressure-sensitive adhesives. Emulsion pressure-sensitive adhesives may include a wide variety of polymeric materials (usually thermoplastic or elastomeric) dispersed in a continuous aqueous phase. The solvent pressure-sensitive adhesives may comprise thermoplastic or elastomeric compositions dissolved in an appropriate aqueous or organic solvent at levels from 1% to 99% solids. All polymer systems in this class are in solutions in the solvent and are not to be confused with emulsions, where the polymer is actually in dispersions. Hot melt pressure-sensitive adhesives may comprise compositions that are capable of being melted and applied to a substrate in its molten state which then cool and solidify relatively quickly. Examples of suitable hot melt adhesives are ethylene-vinyl acetate (EVA) or rubber-based hot melt pressure-sensitive adhesives.

A separate release liner may be provided to cover the pressure-sensitive adhesive. The release liner may be polyethylene or paper or any suitable equivalent thereof that will provide protection. The release liner may be treated on its surface which contacts the fastener so that it has moderate adhesive attachment to the fastener. For instance, a silicone treatment has been found to provide this moderate adhesive attachment with pressure sensitive adhesives.

One or more fasteners 45 may be disposed at or adjacent to each of the first and second lateral edges (12, 13) of the absorbent pad 11 on the topsheet 14 or the backsheet 15. One or more fasteners 42 may be disposed at or adjacent to each of the first lateral edge (41, 41') of the wing (40, 40'). The one or more fasteners 42 may be disposed outboard of each of the first and second lateral edges (41, 43) of the wing 40. For instance, the one or more fasteners 42 may be disposed on a fastening portion 444 of a tape tab 440 as shown in Fig. 13.

A fastener (42, 45) may be provided on a tape tab. The tape tab may be permanently attached to the first and/or second lateral edge (12, 13) of the absorbent pad 11, e.g. on the topsheet 14 or backsheet 15. A portion of the tape tab may extend laterally outboard beyond the first and/or second lateral edge (12, 13) of the absorbent pad 11. The fastener, such as a hook fastener or a pressure sensitive adhesive may be provided on the portion of the tape tab extending outboard the first and/or second lateral edge (12, 13) on the absorbent pad 11.

The tape tab may be permanently attached to the first lateral edge (41, 41') of the one or more wings (40, 40'). A portion of the tape tab may extend laterally outboard beyond the first lateral edge (41, 41') of the one or more wings (40, 40'). The fastener, such as a hook fastener or a pressure sensitive adhesive may be provided on the portion of the tape tab extending outboard the first lateral edge (41, 41') of the one or more wings (40, 40').

Referring now to Fig. 13, an example of a tape tab 440 is depicted. A tape tab may 440 have at least three elements, which are a front surface portion 441, a back surface portion 442, and the fastening portion 444. The tape tab may be attached to the first lateral edge 41 of the wing 40 by the front and back surface portions (441, 442), but it does not need to be. The front surface portion 441 is that portion of the tape tab which is attached to a front surface of the one or more wings 40 with an attachment mean. The back surface portion 442 is that portion of the tape tab which is attached to the back surface of the one or more wings 40 with an attachment mean. The fastening portion 444 is the portion of the tape tab 42 outboard the first and/or second lateral edge (41, 43) of the wing 40. The fastening portion 444 may comprise either a mechanical fastener, e.g. a patch of hook fasteners or a chemical fastener such as a pressure-sensitive adhesive which is able to be releasably engaged to one of the first or second lateral edge (12, 13) of the absorbent pad 11. The tape tab may be provided with a separate release line 443.

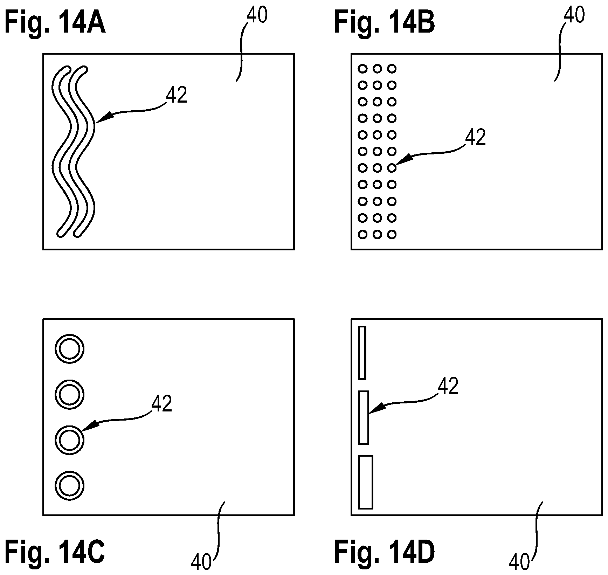

One or more fasteners may have a pattern selected from the group consisting of stripes, wavy pattern, dots, circles and combinations thereof. Various suitable pattern configurations for the one or more fasteners can be contemplated as illustrated in Figs. 14A-14D. The pattern of one or more fasteners may be used as a targeting indicia component in order to indicate, facilitate and/or compel correct positioning and engagement of the one or more wings 40 with the absorbent pad 11.

The one or more fasteners 42 may be continuous or discontinuous stripes at the first and/or second lateral edge (12, 13) of the absorbent pad 11 and/or the one or more wings 40.

The one or more wings 40 may comprise a continuous stripe of hook fasteners or continuous strip of pressure-sensitive adhesive disposed at or adjacent the first and/or second lateral edge (41, 43) of the one or more wings 40.

A package for forming the bed protection device 10 according to the present invention is provided and comprises:
- One or more absorbent pads 11, each having a first and second lateral edges (12, 13) and comprising a liquid permeable topsheet 14, a liquid impermeable backsheet 15 and an absorbent layer 28 located between the topsheet 14 and the backsheet 15,
- One or more wings (40, 40'), each having a first and a second lateral edges (41, 43), the one or more wings (40) being made of a material selected from the group consisting of a woven fabric, a nonwoven web, a film-nonwoven fabric laminate, a tissue and combinations thereof
- One or more fasteners (45) disposing at or adjacent to each of the first and second lateral edges (12, 13) of the absorbent pad 11 on the topsheet 14 or the backsheet 15 and/or one or more fasteners (42, 44, 44') disposing at or adjacent to one or both of the first and second lateral edges (41, 43) of the one or more wings (40, 40').

The one or more wings (40, 40') are able to be releasably engaged with the absorbent pad 11 at or adjacent to the first and/or second lateral edge (12, 13) of the absorbent pad 11 on the topsheet 14 or the backsheet 15 by the one or more fasteners (42, 44, 44', 45).

The package may comprise a ratio between a wing 40 and an absorbent pad 11 of at least 1:2, such as 1:2 to 1:20 or 1:2 to 1:10. For instance, the package may comprise two wings 40 and ten absorbent pads 11.

The package may comprise a first type of absorbent pad having a first type of absorbent layer and a second type of absorbent pad having a second type of absorbent layer. The first type of absorbent layer differs from the second type of absorbent layer. The first and second absorbent layer may have different amount of absorbent material such that the first type and second type of absorbent layer may have different absorption capacities. Hence, the package may comprise different types of absorbent pad for specific usage conditions. In hospitals, some adult incontinent persons may need a bed protection device 10 comprising an absorbent pad 11 having a relatively high amount of absorbent material 60, especially when these adult incontinent persons are subjected to urinate more frequently than a person in a good health. Different types of absorbent pad having different absorption capacities may be useful when it is required to place a bed protection device 10 beneath an adult incontinent person or baby for a long time. This is the case in hospitals when the bed cannot be changed as often as desirable because there are not enough staff members available.

Different types of bed protection device 10 may also be useful for toddlers on toilet-training program. Hence, the consumer with a single package is able to use the appropriate absorbent pads at the appropriate time. The package of the present invention avoids the consumer purchasing a package of each type of absorbent pad. The package of the present invention does also not require excess storage space.

The ratio of the different types of absorbent pads within the single package may be any suitable ratio. For example, the first type and the second type of absorbent pad may be present in a ratio between 12:1 and 3:1. The first type of absorbent pad may have a first type of absorbent layer with a higher amount of absorbent material than the second type of absorbent layer of the second type of absorbent pad. The first type of absorbent pad may be thus used in a bed protection device to be place beneath a person for a long time, e.g. overnight.

The package may comprise a plurality of absorbent pads. Each absorbent pad may have a size. The size of each absorbent pad may differ from each other. The package may comprise a plurality of wings. Each wing may have a size. The size of each wing may differ from each other.

The package can thus provide flexibility for the caregiver to place the right size of the absorbent pad with the right size of the one or more wings according to the size of the bed, or size of the wearer (from an adult or a baby), or size of the surface to be isolated from any contaminations with the liquid bodily exudates.

### Example:

The following bed protection device according to the invention was prepared: An absorbent pad having as a dimension 75 cm x 65 cm was prepared with a 15 gsm nonwoven topsheet having a dimension of 75 cm x 65 cm, a 18 gsm polyethylene backsheet having a dimension of 75 cm x 65 cm and an absorbent layer comprising 60 gsm cellulosic fibers. The absorbent layer has a dimension of 60 cm x 60 cm. A first and second wing each having a dimension of 65 cm x 45 cm and made of a 25 gsm nonwoven web comprises at each first lateral edge a continuous stripe of hook fasteners.

## Claims

1. Bed protection device (10) comprising:
- an absorbent pad (11) having a first and second lateral edges (12, 13);
wherein the absorbent pad (11) comprises a liquid permeable topsheet (14), a liquid impermeable backsheet (15) and an absorbent layer (28) located between the topsheet (14) and the backsheet (15);
- at least one wing (40), wherein the one or more wings (40) is made of a material selected from the group consisting of a woven fabric, a nonwoven web, a film-nonwoven fabric laminate, a tissue and combinations thereof, wherein the one or more wings (40) have a first and a second lateral edges (41, 43);
**characterized in that** one or more fasteners (45) are disposed at or adjacent to each of the first and second lateral edges (12, 13) of the absorbent pad (11) on the topsheet (14) or the backsheet (15) and/or one or more fasteners (42) are disposed at or adjacent to one or both of the first and second lateral edges (41, 43) of the one or more wings (40);
such that the one or more wings (42, 45) are able to be releasably engaged at or adjacent to said lateral edges with the absorbent pad (11) at or adjacent to the first and/or second lateral edge (12, 13) of the absorbent pad (11) on the topsheet (14) or the backsheet (15) by the one or more fasteners (42, 45).

2. Bed protection device (10) according to claim 1 wherein said at least one wing comprises first and second wings (40, 40'), wherein one or more fasteners (42) disposed at or adjacent to the first lateral edge (41) of the first wing (40) are able to be releasably engaged with the absorbent pad (11) at or adjacent to the first lateral edge (12) of the absorbent pad (11) on the topsheet (14) or the backsheet (15); and
wherein one or more fasteners (42) disposed at or adjacent to the first lateral edge (41') of the second wing (40') are able to be releasably engaged with the absorbent pad (11) at or adjacent to the second lateral edge (13) of the absorbent pad (11) on the topsheet (14) or the backsheet (15).

3. Bed protection device (10) according to claim 1 wherein said at least one wing comprises first and second pairs of wings, wherein one or more fasteners disposed at or adjacent to the first lateral edge of the first wings of the respective first and second pairs of wings are able to be releasably engaged with the absorbent pad (11) at or adjacent to the first lateral edge (12) of the absorbent pad (11) on the topsheet (14) or the backsheet (15); and
wherein one or more fasteners disposed at or adjacent to the first lateral edge of the second wings of the respective first and second pairs of wings are able to be releasably engaged with the absorbent pad (11) at or adjacent to the second lateral edge (13) of the absorbent pad (11) on the topsheet (14) or the backsheet (15).

4. Bed protection device (10) according to claims 2 or 3 wherein one or more fasteners (44) disposed at or adjacent to the second lateral edge (43) of the first wing (40) are able to be releasably engaged with one or more fasteners (44') disposed at or adjacent to the second lateral edge (43') of the second wing (40').

5. Bed protection device (10) according to any of the claims 2-4 wherein one or more fasteners (45) are disposed at or adjacent to each of the first and second lateral edges (12, 13) of the absorbent pad (10);
such that the first wing (40) is able to be releasably engaged with the absorbent pad (11) at or adjacent to the first lateral edge (12) of the absorbent pad (11); and
the second wing (40') is able to be releasably engaged with the absorbent pad (11) at or adjacent to the second lateral edge (13) of the absorbent pad (11).

6. Bed protection device (10) according to claim 1 comprising only one wing (40) wherein one or more fasteners (42) disposed at or adjacent to the first lateral edge (41) of the only one wing (40) is able to be releasably engaged with the absorbent pad (11) at or adjacent to the first lateral edge (12) of the absorbent pad (11); and
wherein one or more fasteners (42) disposed at or adjacent to the second lateral edge (41') of the only one wing (40) is able to be releasably engaged with the absorbent pad (11) at or adjacent to the second lateral edge (13) of the absorbent pad (11).

7. Bed protection device (10) according to claim 6 wherein one or more fasteners (45) are disposed at or adjacent to each of the first and second lateral edges (12, 13) of the absorbent pad (11);
such that the only one wing (40) is able to be releasably engaged with the absorbent pad (11) at or adjacent to the first and second lateral edges (12, 13) of the absorbent pad (11).

8. Bed protection device (10) according to claims 5 and 7 wherein one or more fasteners (45) on any of the first and second lateral edges (12, 13) of the absorbent pad (11) correspond to one or more fasteners (42) which are disposed at or adjacent to the first or second lateral edges (41, 41') of the one or more wings (40).

9. Bed protection device (10) according to any preceding claims wherein the one or more fasteners (42, 44, 44', 45) are mechanical or chemical fasteners.

10. Bed protection device (10) according to claim 9 wherein the mechanical fasteners are selected from the group consisting of hook fastener, loop fastener, interlocking fastener and combinations thereof, or wherein the chemical fasteners are pressure sensitive adhesives.

11. Bed protection device (10) according to any of the preceding claims wherein one or more fasteners (42, 44, 44', 45) are provided on a tape tab.

12. Bed protection device according to any of the preceding claims wherein one or more fasteners (42, 44, 44', 45) have a pattern selected from the group consisting of stripes, wavy pattern, dots, circles and combinations thereof.

13. Bed protection device (10) according to any the preceding claims wherein the one or more wings (40) comprises a continuous stripe of hook fasteners disposed at or adjacent the first and/or second lateral edge of the one or more wings.

14. A package for forming the bed protection device according to claim 1 comprising:
- One or more absorbent pads (11), each having a first and second lateral edges (12, 13) and comprising a liquid permeable topsheet (14), a liquid impermeable backsheet (15) and an absorbent layer (28) located between the topsheet (14) and the backsheet (15);
- One or more wings (40), each having a first and a second lateral edges (41, 43), wherein the one or more wings (40) is made of a material selected from the group consisting of a woven fabric, a nonwoven web, a film-nonwoven fabric laminate, a tissue and combinations thereof;
- One or more fasteners (45) disposed at or adjacent to each of the first and second lateral edges (12, 13) of the absorbent pad (11) on the topsheet (14) or the backsheet (15) and/or one or more fasteners (42, 44, 44') disposed at or adjacent to one or both of the first and second lateral edges (41, 43) of the one or more wings (40);
such that the one or more wings (40) are able to be releasably engaged with the absorbent pad (11) at or adjacent to the first and/or second lateral edge (12, 13) of the absorbent pad (11) on the topsheet (14) or the backsheet (15) by the one or more fasteners (42, 44, 44', 45).

15. The package according to claim 14 wherein the package comprises a first type of absorbent pad having a first type of absorbent layer and a second type of absorbent pad having a second type of absorbent layer and wherein the first type of absorbent layer differs from the second type of absorbent layer.

16. The package according to claim 14 wherein the package comprises a plurality of absorbent pads, each absorbent pad having a size, wherein the size of each absorbent pad differs from each other and/or wherein the package comprises a plurality of wings, each wing having a size, wherein the size of each wing differs from each other.

## Patentansprüche

1. Bettschutzvorrichtung (10), umfassend:
- eine Absorptionseinlage (11) mit einem ersten und einem zweiten Seitenrand (12, 13);
wobei die Absorptionseinlage (11) eine flüssigkeitsdurchlässige Oberschicht (14), eine flüssigkeitsundurchlässige Unterschicht (15) und eine Absorptionsschicht (28) umfasst, die zwischen der Oberschicht (14) und der Unterschicht (15) angeordnet ist;
- mindestens einen Flügel (40), wobei der eine oder die mehreren Flügel (40) aus einem Material hergestellt sind, das ausgewählt ist aus der Gruppe bestehend aus einem Gewebe, einer Vliesbahn, einem Folien-Vlies-Stofflaminat, einem Tissue und Kombinationen daraus, wobei der eine oder die mehreren Flügel (40) einen ersten und einen zweiten Seitenrand (41, 43) aufweisen;
**dadurch gekennzeichnet, dass** ein oder mehrere Befestigungsmittel (45) an oder benachbart zu dem ersten und an oder benachbart zu dem zweiten Seitenrand (12, 13) der Absorptionseinlagen (11) auf der Oberschicht (14) oder der Unterschicht (15) angeordnet sind, und/oder dass ein oder mehrere Befestigungsmittel (42) an oder benachbart zu dem ersten und/oder an oder benachbart zu dem zweiten Seitenrand (41, 43) des einen Flügels oder der mehreren Flügel (40) angeordnet sind;
sodass der eine oder die mehreren Flügel (42, 45) durch das eine oder die mehreren Befestigungsmittel (42, 45) an oder benachbart zu den Seitenrändern mit der Absorptionseinlage (11) an oder benachbart zu dem ersten und/oder dem zweiten Seitenrand (12, 13) der Absorptionseinlage (11) an der Oberschicht (14) oder der Unterschicht (15) lösbar in Eingriff gelangen können.

2. Bettschutzvorrichtung (10) nach Anspruch 1, wobei der mindestens eine Flügel einen ersten und einen zweiten Flügel (40, 40') umfasst, wobei das eine oder die mehreren an oder benachbart zu dem ersten Seitenrand (41) des ersten Flügels (40) angeordneten Befestigungsmittel (42) mit der Absorptionseinlage (11) an oder benachbart zu dem ersten Seitenrand (12) der Absorptionseinlage (11) an der Oberschicht (14) oder der Unterschicht (15) lösbar in Eingriff gelangen können; und
wobei ein oder mehrere an oder benachbart zu dem ersten Seitenrand (41') des zweiten Flügels (40') angeordnete Befestigungsmittel (42) mit der Absorptionseinlage (11) an oder benachbart zu dem zweiten Seitenrand (13) der Absorptionseinlage (11) an der Oberschicht (14) oder der Unterschicht (15) lösbar in Eingriff gelangen können.

3. Bettschutzvorrichtung (10) nach Anspruch 1, wobei der mindestens eine Flügel erste und zweite Paare von Flügeln umfasst, wobei ein oder mehrere an oder benachbart zu dem ersten Seitenrand der ersten Flügel des ersten bzw. des zweiten Flügelpaars angeordnete Befestigungsmittel mit der Absorptionseinlage (11) an oder benachbart zu dem ersten Seitenrand (12) der Absorptionseinlage (11) an der Oberschicht (14) oder der Unterschicht (15) lösbar in Eingriff gelangen können; und
wobei ein oder mehrere an oder benachbart zu dem ersten Seitenrand der zweiten Flügel des ersten bzw. des zweiten Flügelpaars angeordnete Befestigungsmittel mit der Absorptionseinlage (11) an oder benachbart zu dem zweiten Seitenrand (13) der Absorptionseinlage (11) an der Oberschicht (14) oder der Unterschicht (15) lösbar in Eingriff gelangen können.

4. Bettschutzvorrichtung (10) nach Anspruch 2 oder 3, wobei ein oder mehrere an oder benachbart zu dem zweiten Seitenrand (43) des ersten Flügels (40) angeordnete Befestigungsmittel (44) mit einem oder mehreren an oder benachbart zu dem zweiten Seitenrand (43') des zweiten Flügels (40') angeordneten Befestigungsmitteln (44') lösbar in Eingriff gelangen können.

5. Bettschutzvorrichtung (10) nach einem der Ansprüche 2 bis 4, wobei ein oder mehrere Befestigungsmittel (45) an oder benachbart zu sowohl dem ersten als auch dem zweiten Seitenrand (12, 13) der Absorptionseinlage (10) angeordnet sind;
sodass der erste Flügel (40) mit der Absorptionseinlage (11) an oder benachbart zu dem ersten Seitenrand (12) der Absorptionseinlage (11) lösbar in Eingriff gelangen kann; und
sodass der zweite Flügel (40') mit der Absorptionseinlage (11) an oder benachbart zu dem zweiten Seitenrand (13) der Absorptionseinlage (11) lösbar in Eingriff gelangen kann.

6. Bettschutzvorrichtung (10) nach Anspruch 1, umfassend nur einen Flügel (40), wobei das eine oder die mehreren an oder benachbart zu dem ersten Seitenrand (41) des nur einen Flügels (40) angeordneten Befestigungsmittel (42) mit der Absorptionseinlage (11) an oder benachbart zu dem ersten Seitenrand (12) der Absorptionseinlage (11) lösbar in Eingriff gelangen können; und
wobei ein oder mehrere an oder benachbart zu dem zweiten Seitenrand (41') des nur einen Flügels (40) angeordnete Befestigungsmittel (42) mit der Absorptionseinlage (11) an oder benachbart zu dem zweiten Seitenrand (13) der Absorptionseinlage (11) lösbar in Eingriff gelangen können.

7. Bettschutzvorrichtung (10) nach einem Anspruch 6, wobei ein oder mehrere Befestigungsmittel (45) an oder benachbart sowohl zu dem ersten als auch dem zweiten Seitenrand (12, 13) der Absorptionseinlage (11) angeordnet sind;
sodass der nur eine Flügel (40) mit der Absorptionseinlage (11) an oder benachbart zu dem ersten und dem zweiten Seitenrand (12, 13) der Absorptionseinlage (11) lösbar in Eingriff gelangen kann.

8. Bettschutzvorrichtung (10) nach Anspruch 5 oder 7, wobei ein oder mehrere Befestigungsmittel (45) an dem ersten und/oder dem zweiten Seitenrand (12, 13) der Absorptionseinlage (11) den ein oder mehreren Befestigungsmitteln (42) entsprechen, die an oder benachbart zu dem ersten oder dem zweiten Seitenrand (41, 41') des einen Flügels oder der mehreren Flügel (40) angeordnet sind.

9. Bettschutzvorrichtung (10) nach einem der vorstehenden Ansprüche, wobei das eine oder die mehreren Befestigungsmittel (42, 44, 44', 45) mechanische oder chemische Befestigungsmittel sind.

10. Bettschutzvorrichtung (10) nach Anspruch 9, wobei die mechanischen Befestigungsmittel ausgewählt sind aus der Gruppe bestehend aus Hakenbefestigung, Schlaufenbefestigung, Verbundbefestigung und Kombinationen daraus, oder wobei die chemischen Befestigungsmittel druckempfindliche Klebemittel sind.

11. Bettschutzvorrichtung (10) nach einem der vorstehenden Ansprüche, wobei das eine oder die mehreren Befestigungsmittel (42, 44, 44', 45) auf einem Bandstreifen bereitgestellt sind.

12. Bettschutzvorrichtung nach einem der vorstehenden Ansprüche, wobei ein oder mehrere Befestigungsmittel (42, 44, 44', 45) ein Muster aufweisen, ausgewählt aus der Gruppe bestehend aus Streifen, Wellenmustern, Punkten, Kreisen und Kombinationen davon.

13. Bettschutzvorrichtung (10) nach einem der vorstehenden Ansprüche, wobei der eine oder die mehreren Flügel (40) einen kontinuierlichen Streifen aus Hakenbefestigern an oder benachbart zu dem ersten und/oder dem zweiten Seitenrand des einen Flügels oder der mehreren Flügel umfassen.

14. Packung zum Bilden der Bettschutzvorrichtung nach Anspruch 1, umfassend:
- eine oder mehrere Absorptionseinlagen (11), jeweils aufweisend einen ersten und einen zweiten Seitenrand (12, 13) und umfassend eine flüssigkeitsdurchlässige Oberschicht (14), eine flüssigkeitsundurchlässige Unterschicht (15) und eine Absorptionsschicht (28), die zwischen der Oberschicht (14) und der Unterschicht (15) angeordnet ist;
- einen oder mehrere Flügel (40), jeweils aufweisend einen ersten und einen zweiten Seitenrand (41, 43), wobei der eine oder die mehreren Flügel (40) aus einem Material hergestellt sind, das ausgewählt ist aus der Gruppe bestehend aus einem Gewebe, einer Vliesbahn, einem Folien-Vlies-Stofflaminat, einem Tissue und Kombinationen davon;
- einen oder mehrere, an oder benachbart zu dem ersten und an oder benachbart zu dem zweiten Seitenrand (12, 13) der Absorptionseinlage (11) auf der Oberschicht (14) oder der Unterschicht (15) angeordnete Befestigungsmittel (45) und/oder einen oder mehrere, an oder benachbart zu dem ersten und/oder an oder benachbart zu dem zweiten Seitenrand (41, 43) des einen Flügels oder der mehreren Flügel (40) angeordnete Befestigungsmittel (42, 44, 44');
sodass der eine oder die mehreren Flügel (40) durch das eine oder die mehreren Befestigungsmittel (42, 44, 44', 45) an oder benachbart zu dem ersten und/oder dem zweiten Seitenrand (12, 13) der Absorptionseinlage (11) an der Oberschicht (14) oder an der Unterschicht (15) mit der Absorptionseinlage (11) lösbar in Eingriff gelangen können.

15. Packung nach Anspruch 14, wobei die Packung einen ersten Typ von Absorptionseinlage mit einem ersten Typ einer Absorptionsschicht und einen zweiten Typ von Absorptionseinlage mit einem zweiten Typ einer Absorptionsschicht umfasst, und wobei sich der erste Typ einer Absorptionsschicht vom zweiten Typ einer Absorptionsschicht unterscheidet.

16. Packung nach Anspruch 14, wobei die Packung eine Vielzahl von Absorptionseinlagen umfasst, wobei jede Absorptionseinlage eine Größe aufweist, wobei die Größen aller Absorptionseinlagen voneinander unterschiedlich sind, und/oder wobei die Packung eine Vielzahl von Flügeln umfasst, wobei jeder Flügel eine Größe aufweist, wobei die Größen aller Flügel voneinander unterschiedlich sind.

## Revendications

1. Dispositif de protection de lit (10) comprenant :
- un tampon absorbant (11) ayant des premier et deuxième bords latéraux (12, 13) ;
dans lequel le tampon absorbant (11) comprend une feuille de dessus perméable aux liquides (14), une feuille de fond imperméable aux liquides (15) et une couche absorbante (28) située entre la feuille de dessus (14) et la feuille de fond (15) ;
- au moins une ailette (40), dans lequel la ou les ailettes (40) sont constituées d'un matériau choisi dans le groupe constitué d'un tissu tissé, une nappe non tissée, un stratifié film-étoffe non tissée, un papier absorbant et leurs combinaisons, dans lequel la ou les ailettes (40) ont des premier et deuxième bords latéraux (41, 43) ;
**caractérisé en ce qu'**un ou plusieurs éléments de fixation (45) sont disposés au niveau de, ou adjacents à, chacun des premier et deuxième bords latéraux (12, 13) du tampon absorbant (11) sur la feuille de dessus (14) ou la feuille de fond (15) et/ou un ou plusieurs éléments de fixation (42) sont disposés au niveau de, ou adjacents à, l'un et/ou l'autre des premier et deuxième bords latéraux (41, 43) de la ou des ailettes (40) ;
de telle sorte que la ou les ailettes (42, 45) sont aptes à être mises en prise de manière libérable au niveau desdits, ou adjacentes auxdits, bords latéraux avec le tampon absorbant (11) au niveau des, ou adjacent aux, premier et/ou deuxième bords latéraux (12, 13) du tampon absorbant (11) sur la feuille de dessus (14) ou la feuille de fond (15) par le ou les éléments de fixation (42, 45).

2. Dispositif de protection de lit (10) selon la revendication 1, dans lequel ladite au moins une ailette comprend des première et deuxième ailettes (40, 40'), dans lequel un ou plusieurs éléments de fixation (42) disposés au niveau du, ou adjacents au, premier bord latéral (41) de la première ailette (40) sont aptes à être mis en prise de manière libérable avec le tampon absorbant (11) au niveau du, ou adjacents au, premier bord latéral (12) du tampon absorbant (11) sur la feuille de dessus (14) ou la feuille de fond (15) ; et
dans lequel un ou plusieurs éléments de fixation (42) disposés au niveau du, ou adjacents au, premier bord latéral (41') de la deuxième ailette (40') sont aptes à être mis en prise de manière libérable avec le tampon absorbant (11) au niveau du, ou adjacents au, deuxième bord latéral (13) du tampon absorbant (11) sur la feuille de dessus (14) ou la feuille de fond (15).

3. Dispositif de protection de lit (10) selon la revendication 1, où ladite au moins une ailette comprend des première et deuxième paires d'ailettes, dans lequel un ou plusieurs éléments de fixation disposés au niveau du, ou adjacents au, premier bord latéral des premières ailettes parmi les première et deuxième paires d'ailettes respectives sont aptes à être mis en prise de manière libérable avec le tampon absorbant (11) au niveau du, ou adjacents au, premier bord latéral (12) du tampon absorbant (11) sur la feuille de dessus (14) ou la feuille de fond (15) ; et
dans lequel un ou plusieurs éléments de fixation disposés au niveau du, ou adjacents au, premier bord latéral des deuxièmes ailettes parmi les première et deuxième paires d'ailettes respectives sont aptes à être mis en prise de manière libérable avec le tampon absorbant (11) au niveau du, ou adjacents au, deuxième bord latéral (13) du tampon absorbant (11) sur la feuille de dessus (14) ou la feuille de fond (15).

4. Dispositif de protection de lit (10) selon les revendications 2 ou 3, dans lequel un ou plusieurs éléments de fixation (44) disposés au niveau du, ou adjacents au, deuxième bord latéral (43) de la première ailette (40) sont aptes à être mis en prise de manière libérable avec un ou plusieurs éléments de fixation (44') disposés au niveau du, ou adjacents au, deuxième bord latéral (43') de la deuxième ailette (40').

5. Dispositif de protection de lit (10) selon l'une quelconque des revendications 2 à 4, dans lequel un ou plusieurs éléments de fixation (45) sont disposés au niveau de, ou adjacents à, chacun des premier et deuxième bords latéraux (12, 13) du tampon absorbant (10) ;
de telle sorte que la première ailette (40) est apte à être mise en prise de manière libérable avec le tampon absorbant (11) au niveau du, ou adjacente au, premier bord latéral (12) du tampon absorbant (11) ; et
la deuxième ailette (40') est apte à être mise en prise de manière libérable avec le tampon absorbant (11) au niveau du, ou adjacente au, deuxième bord latéral (13) du tampon absorbant (11).

6. Dispositif de protection de lit (10) selon la revendication 1, comprenant une seule ailette (40), dans lequel un ou plusieurs éléments de fixation (42) disposés au niveau du, ou adjacents au, premier bord latéral (41) de la seule ailette (40) sont aptes à être mis en prise de manière libérable avec le tampon absorbant (11) au niveau du, ou adjacents au, premier bord latéral (12) du tampon absorbant (11) ; et
dans lequel un ou plusieurs éléments de fixation (42) disposés au niveau du, ou adjacents au, deuxième bord latéral (41') de la seule ailette (40) sont aptes à être mis en prise de manière libérable avec le tampon absorbant (11) au niveau du, ou adjacents au, deuxième bord latéral (13) du tampon absorbant (11).

7. Dispositif de protection de lit (10) selon la revendication 6, dans lequel un ou plusieurs éléments de fixation (45) sont disposés au niveau de, ou adjacents à, chacun des premier et deuxième bords latéraux (12, 13) du tampon absorbant (11) ;
de telle sorte que la seule ailette (40) est apte à être mise en prise de manière libérable avec le tampon absorbant (11) au niveau des, ou adjacente aux, premier et deuxième bords latéraux (12, 13) du tampon absorbant (11).

8. Dispositif de protection de lit (10) selon les revendications 5 et 7, dans lequel un ou plusieurs éléments de fixation (45) sur n'importe lequel des premier et deuxième bords latéraux (12, 13) du tampon absorbant (11) correspondent auxdits un ou plusieurs éléments de fixation (42) qui sont disposés au niveau des, ou adjacents aux, premier ou deuxième bords latéraux (41, 41') de la ou des ailettes (40).

9. Dispositif de protection de lit (10) selon l'une quelconque des revendications précédentes, dans lequel le ou les éléments de fixation (42, 44, 44', 45) sont des éléments de fixation mécaniques ou chimiques.

10. Dispositif de protection de lit (10) selon la revendication 9, dans lequel les éléments de fixation mécaniques sont choisis dans le groupe constitué d'élément de fixation à crochet, élément de fixation à boucle, élément de fixation à enclenchement réciproque et leurs combinaisons, ou dans lequel l'élément de fixation chimique est constitué d'adhésifs sensibles à la pression.

11. Dispositif de protection de lit (10) selon l'une quelconque des revendications précédentes, dans lequel un ou plusieurs éléments de fixation (42, 44, 44', 45) sont fournis sur une languette de ruban.

12. Dispositif de protection de lit selon l'une quelconque des revendications précédentes, dans lequel un ou plusieurs éléments de fixation (42, 44, 44', 45) ont un motif choisi dans le groupe constitué de bandes, motifs ondulés, points, cercles et leurs combinaisons.

13. Dispositif de protection de lit (10) selon l'une quelconque des revendications précédentes, dans lequel la ou les ailettes (40) comprennent une bande continue d'éléments de fixation à crochet disposés au niveau des, ou adjacents aux, premier et/ou deuxième bords latéraux de la ou des ailettes.

14. Conditionnement pour former le dispositif de protection de lit selon la revendication 1, comprenant :
- un ou plusieurs tampons absorbants (11), ayant chacun des premier et deuxième bords latéraux (12, 13) et comprenant une feuille de dessus perméable aux liquides (14), une feuille de fond imperméable aux liquides (15) et une couche absorbante (28) située entre la feuille de dessus (14) et la feuille de fond (15) ;
- une ou plusieurs ailettes (40), ayant chacune des premier et deuxième bords latéraux (41, 43), dans lequel la ou les ailettes (40) sont constituées d'un matériau choisi dans le groupe constitué d'un tissu tissé, une nappe non tissée, un stratifié film-étoffe non tissée, un papier absorbant et leurs combinaisons ;
- un ou plusieurs éléments de fixation (45) disposés au niveau de, ou adjacents à, chacun des premier et deuxième bords latéraux (12, 13) du tampon absorbant (11) sur la feuille de dessus (14) ou la feuille de fond (15) et/ou un ou plusieurs éléments de fixation (42, 44, 44') disposés au niveau de, ou adjacents à, l'un et/ou l'autre des premier et deuxième bords latéraux (41, 43) de la ou des ailettes (40) ;
de telle sorte que la ou les ailettes (40) sont aptes à être mises en prise de manière libérable avec le tampon absorbant (11) au niveau des, ou adjacents aux, premier et/ou deuxième bords latéraux (12, 13) du tampon absorbant (11) sur la feuille de dessus (14) ou la feuille de fond (15) par le ou les éléments de fixation (42, 44, 44', 45).

15. Conditionnement selon la revendication 14, où le conditionnement comprend un premier type de tampon absorbant ayant un premier type de couche absorbante et un deuxième type de tampon absorbant ayant un deuxième type de couche absorbante et dans lequel le premier type de couche absorbante diffère du deuxième type de couche absorbante.

16. Conditionnement selon la revendication 14, où le conditionnement comprend une pluralité de tampons absorbants, chaque tampon absorbant ayant une taille, dans lequel la taille de chaque tampon absorbant diffère mutuellement et/ou où le conditionnement comprend une pluralité d'ailettes, chaque ailette ayant une taille, dans lequel la taille de chaque ailette diffère mutuellement.
